# EUROPEAN PATENT APPLICATION

(11) **EP 1 557 161 A1**
(43) Date of publication of application: **27.07.2005**
(21) Application number: 05250363.8
(22) Date of filing: 25.01.2005
(51) Int. Cl.: A61K 7/50

(54) **Method for cleansing sensitive skin using an alkanolamine**

(30) Priority: 26.01.2004 US 764856
(71) Applicant: Johnson & Johnson Consumer Companies, Inc., Skillman, New Jersey 08558-9418 (US)
(72) Inventor: Cole, Curtis A., Ringoes, NJ 08551 (US); Aleles, Margaret A., Gladstone, NJ 07934 (US); Lukenbach, Elvin R., Flemington, NJ 08822 (US); Skover, Greg, Princeton, NJ 08540 (US); Barkovic, Sylvia, Long Beach, CA 90814 (US); Shah, Snehal, Cerritos, CA 90703 (US); Campoblanco, Blanca, Downey, CA 90240 (US); Appa, Yohini, Torrance, CA 90501 (US)
(74) Representative: Mercer, Christopher Paul

(57) **Abstract**

The invention relates to a method of simultaneously cleansing the skin and reducing skin sensitivity and/or skin reactivity. The method comprises topically applying a skin cleanser composition comprising: (a) an effective amount of an alkanolamine the formula: 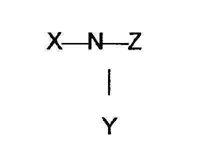 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group; (b) a cleansing surfactant; and (c) water. In another embodiment, the invention relates to a method for ameliorating redness or inflammation of mammalian skin by topically applying the above skin cleanser composition.

## Description

### FIELD OF THE INVENTION

This invention relates to skin cleansers that are non-irritating to the skin which can be used to simultaneously clean and reduce inflammation of the skin.

### BACKGROUND OF THE INVENTION

Self-perceived and clinically diagnosed sensitive skin, i.e., skin that is easily irritated by topical ingredients at concentrations lower than evident in a normal skin individual, and reactive skin, i.e., skin that is easily irritated by topical ingredients where the reaction can extend beyond the application site and can also be triggered by emotional stress and changes in temperature and humidity is on the rise. The result is more individuals suffering from rosacea, skin rashes, skin redness, blotchy skin, uneven skin coloration and vascularity and other inflammatory conditions generating a need for mild facial products. Conventional skin cleansers contain surfactants that remove dirt, oil and surface elements from the skin but are known to be irritating to the skin. Consumers with sensitive or reactive skin often sacrifice product aesthetics in order not to irritate or inflame their sensitive skin. Accordingly, there is a need for a skin cleansing composition that does not cause inflammation of the skin.

Copending U.S. Patent Application Serial No. 09/677,737, filed October 2, 2002 relates to a method for ameliorating redness or inflammation of mammalian skin comprising topically applying a composition comprising an effective amount of at least one compound selected from an alkanolamine, tyrosine, or mixtures thereof and a cosmetically acceptable carrier. Skin cleanser compositions are not disclosed.

It has surprisingly been discovered that by adding an alkanolamine to skin cleanser compositions containing surfactant, the cleanser becomes milder. This is particularly surprising since alkanolamines are caustic amine bases can be irritating chemicals. It has also been discovered that pre-existing skin inflammations, such as, rosacea and atopic dermatitis, can even be reduced by using a skin cleanser composition containing surfactant and an alkanolamine.

### SUMMARY OF INVENTION

Accordingly, the invention relates to a method of simultaneously cleansing the skin and reducing skin sensitivity and reactiveness. The method comprises topically applying a skin cleanser composition comprising:
(a) an effective amount of an alkanolamine the formula: 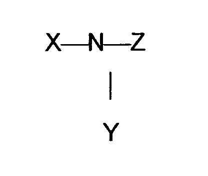 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group; and
(b) a cleansing surfactant; and
(c) water.

In another embodiment, the invention relates to a method for ameliorating redness or inflammation of mammalian skin by topically applying a composition comprising:
(a) an effective amount of at least one alkanolamine;
(b) a cleansing surfactant; and
(c) water;
wherein said alkanolamine has the following general formula: 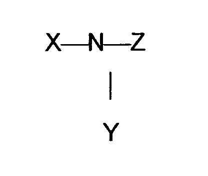 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

### BRIEF DESCRIPTION OF THE DRAWINGS

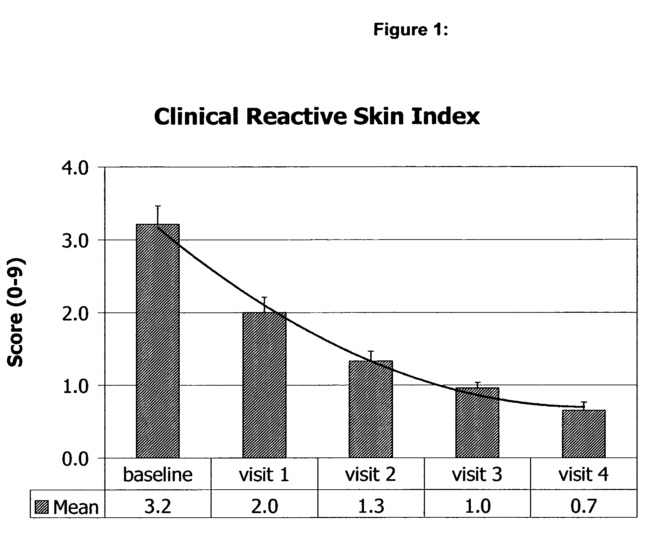

Figure 1 is a bar graph demonstrating the clinical grading scores of the quality of the skin as assessed by a dermatologist after application of the cleanser of Example 1.

### DETAILED DESCRIPTION OF THE INVENTION

The invention relates to a method of simultaneously cleansing the skin and reducing skin sensitivity and/or skin reactivity. As discussed above, sensitive skin is skin that is easily irritated by topical ingredients at concentrations lower than evident in a normal skin individual. Typical reactions include inflammation, redness or rash at the site of the product application. Reactive skin is skin that is also easily irritated by topical ingredients but the reaction can extend beyond the application site. Reactive skin can also be triggered by emotional stress and changes in temperature and humidity. Reactions include, inflammation, redness, rash, blotchy, uneven skin coloration and vascularity.

The alkanolamine used in the methods according to the invention has the following general formula: 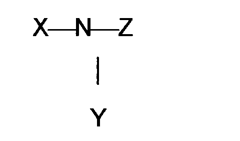 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

In a preferred embodiment the alkanolamine is selected from the group consisting of ethylaminoethanol, methylaminoethanol, dimethylaminoethanol, isopropanolamine, triethanolamine, isopropanoldimethylamine, ethylethanolamine, 2-butanolamine, choline and serine. More preferably, the alkanolamine is dimethylaminoethanol (DMAE). The cleansing compositions used in the methods according to the invention preferably contain from about 0.1 about 10% by weight of the alkanolamine, more preferably, from about 0.1 to about 5% and, most preferably, from about 1 to about 3% by weight based on the total composition.

The alkanolamine salt can be made by techniques known in the art by reacting the desired alkanolamine with an appropriate acid under conditions sufficient to form the salt. The salts can be formed *in situ* or prior to formulating. Generally, when at least one of W, X, Y, or Z is hydrogen the alkanolamine salt can be prepared *in situ*.

In a preferred embodiment, the alkanoloamine salt is an acid salt of monomethylaminoethanol, dimethylaminoethanol, trimethylammonioethanol hydroxide, isopropanolamine, triethanolamine, isoropanoldimethylamine, ethylethanolamine, 2-butanolamine, choline, serine, and copolymers thereof.

Suitable acids for use in the preparation of the alkanolamine salts according to the invention include any organic acid known to be useful in skin care compositions. In a preferred embodiment, at least one of the acids is an alpha hydroxy acid, such as taught for example in U.S. Patent No. 5,856,357, the disclosure of which is hereby incorporated by reference. Particularly preferred is a mixture of at least two of glycolic acid, malic acid and citric acids. In a most preferred embodiment, the acid is a combination of glycolic acid and either malic or citric acid. In situations where pH stability is a particular concern, e.g., long term storage, a particularly preferred embodiment is when the acid is a mixture of citric and glycolic acid. Preferably, the ratio of malic or citric acid to glycolic acid ranges from about 1:1 to about 1:5, more preferably, from about 1:1 to about 1:3.

In a preferred embodiment, the compositions used in the methods of the invention contain a pH buffering agent. Preferably, the amount of buffering agent should be that which would result in compositions having a pH ranging from about 4.0 to about 9.0, more preferably from about 5.5 to about 8.5. The buffering agent can be any of the known buffering agents commonly found in cosmetic compositions provided that they are physically and chemically stable with the other ingredients of the composition. Suitable buffering agents include but are not intended to be restricted to organic acids such as citric acid, malic acid, and glycolic acid.

The cleansing compositions according to the invention can comprise additional ingredients commonly found in skin care compositions, such as for example, emollients, skin conditioning agents, emulsifying agents, humectants, preservatives, antioxidants, perfumes, chelating agents, etc., provided that they are physically and chemically compatible with the other components of the composition. Notably useful is the incorporation of vitamin A and vitamin A derivatives, including but not restricted to retinol, retinyl palmitate, retinoic acid, retinal, and retinyl propionate.

Examples of suitable preservatives for use in the compositions of the invention include the C₁-C₄ alkyl parabens and phenoxyethanol. Generally, the preservative is present in an amount ranging from about 0.5 to about 2.0, preferably about 1.0 to about 1.5, weight percent based on the total composition. In a preferred embodiment, the preservative is mixture of from about 0.2 to about 0.5 weight percent methylparaben, from about 0.2 to about 5.0 weight percent propylparaben and from about 0.05 to about 0.10 weight percent butylparaben. A particularly preferred commercially available preservative that may be used in the skin care composition according to this invention is PHENONIP TM which is a practically colorless, viscous, liquid mixture of phenoxyethanol, methylparaben, ethylparaben, propylparaben, and butylparaben available from Nipa Laboratories, Inc., Wilmington, Del.

Preferably, antioxidants should be present in the compositions according to the invention. Suitable antioxidants include butylated hydroxy toluene (BHT), ascorbyl palmitate, butylated hydroxyanisole (BHA), phenyl-α-naphthylamine, hydroquinone, propyl gallate, nordihydroquiaretic acid, vitamin E or derivatives of vitamin E, vitamin C or derivatives of vitamin C, calcium pantothenic, green tea extracts and mixed polyphenosls, and mixtures thereof of the above. When utilized the antioxidant can be present in an amount ranging from about 0.02 to about 0.5% by weight, more preferably from about 0.002 to about 0.1% by weight of the total composition.

Emollients which can be included in the compositions of the invention function by their ability to remain on the skin surface or in the stratum corneum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, cetyl alcohol, octyl hydroxystearate, dimethicone, and combinations thereof. Cetyl alcohol, octyl hydroxystearate, dimethicone, and combinations thereof are preferred. When utilized, the emollient can be present in an amount from about 0.01 to about 5, preferably from about 1 to about 4 percent by weight based on the total composition.

Polyhydric alcohols can be utilized as humectants in the compositions of the invention. The humectants aid in increasing the effectiveness of the emollient, reduce scaling, stimulate removal of built-up scale and improve skin feel. Suitable polyhydric alcohols include, but are not limited to, glycerol (also known as glycerin), polyalkylene glycols, alkylene polyols and their derivatives, including butylene glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 10, preferably from about 1 to about 7 percent by weight, based on the total weight of the composition.

Any fragrance may be added to the compositions of the invention for aesthetic purposes. Suitable fragrances include, but are not limited to, eucalyptus oil, camphor synthetic, peppermint oil, clove oil, lavender, chamomile and the like. When utilized, fragrances are present in an amount from about 0.05 to about 0.5, preferably from about 0.1 to about 0.3 percent by weight, based on the total weight of the composition.

In certain aspects of this invention, the compositions should include a chelating agent. Chelating agents which are useful in the compositions of the present invention include ethylenediamine tetra acetic acid (EDTA) and derivatives and salts thereof, dihydroxyethyl glycine, tartaric acid, and mixtures thereof. The chelating agents should be utilized in a stabilizing effective amount and may range from about 0.01 to about 2% based on the weight of the total composition, preferably from about 0.05 to about 1%. Most preferably, the chelating agent should be EDTA.

In one embodiment, the cleansing composition of the invention is substantially free of vitamin C and derivatives thereof such as ascorbyl palmitate, sodium ascorbyl phosphate, magnesium ascorbyl phosphate and similar type molecules. By "substantially free" it is meant less than 0.5% by weight of the cleansing composition. In another embodiment, the cleansing composition is free from vitamin C and derivatives thereof.

The skin cleanser may be in the form of an oil-in-water emulsion, a water-in-oil emulsion, dispersion, microemulsion, or a lamellar (liquid crystalline) composition. The skin cleanser of the present invention comprises at least one cleansing surfactant including foaming surfactants and non-foaming surfactants. Suitable surfactants include non-ionic, cationic, amphoteric, or anionic surfactants; nonionic surfactants are preferred. By "foaming," it is meant that the surfactant, when used with the composition of the present invention, has a column height of foam greater than about 20 mm as determined by the Ross-Miles Foam Generation Test. See 18 (I.) Oil & Soap 99 - 102 (1941) ("Ross-Miles Test"), which is incorporated by reference herein. As used herein, the term "amphoteric" shall mean: 1) molecules that contain both acidic and basic sites such as, for example, an amino acid containing both amino (basic) and acid (e.g., carboxylic acid, acidic) functional groups; or 2) zwitterionic molecules which possess both positive and negative charges within the same molecule. The charges of the latter may be either dependent on or independent of the pH of the composition. Examples of zwitterionic materials include, but are not limited to, alkyl betaines and amidoalkyl betaines. Examples of suitable and preferred foaming and non-foaming surfactants may be found in copending application Serial No. 09/604,563, filed June 27, 2000, which is incorporated by reference in its entirety herein. Specific examples of suitable foaming surfactants include sodium cocoyl sarcosinate, decyl glucoside, lauryl glucoside, ammonium laureth sulfate, cocoamidopropyl betaine, lauryl betaine, sodium cocoamphoacetate, and mixtures thereof. Generally, the foaming surfactant should be present in an amount to provide effective cleansing properties. In one embodiment, the foaming surfactant is present in an amount ranging from about 10 to about 40% by weight of the cleansing composition. For purposes of economy and for low intensity of action to reduce drying and irritancy for facial use, in one embodiment, the foaming surfactant is present at from about 5 to about 20% by weight of the cleansing composition.

Examples of suitable non-foaming surfactants include non-foaming nonionic surfactants such as sucrose esters, e.g., sucrose cocoate, sucrose stearate and mixtures thereof, with sucrose cocoate being preferred. By "non-foaming," it is meant that the surfactant, when used with the composition of the present invention, has a column height of less than about 20 mm as determined by the Ross-Miles Test. A preferred combination of non-foaming surfactant and hydrophilic components include, based upon the total weight percent of the skin cleanser, from about 0.1 percent to about 5.0 percent of hexylene glycol, from about 0.5 percent to about 3.0 percent of sucrose cocoate non foaming surfactant, and from about 0.5 percent to about 3.0 percent of polyoxyethylene-6 caprylic/capric triglyceride. An example of a suitable cleaning enhancer include a mixture of sorbitan stearate and sucrose cocoate available from Uniqema under the tradename, "Arlatone 2121." Preferably, the skin cleanser contains, based upon the total weight of the skin cleanser, no more than about 6%, and preferably 5%, of the non-foaming surfactant.

The skin cleanser of the present invention may also optionally contain a cleaning enhancer in the form of a nonionic emulsifier. Examples of suitable nonionic emulsifiers include isocetheth-20, oleth-2, mixture of PEG-40 hydrogenated castor oil and trideceth-9 available from Dragoco Inc. under the tradename, "Dragoco Solubilizer 2/014160," Poloxamer 184, laureth-4, sorbitan trioleate, polyoxyethylene-(2) oleyl ether, PEG-20 methyl glucose sesquistereate, methyl glucose dioleate, sorbitan stearate, cetearyl glucoside, glyceryl oleate, trideceth-9, polyethylene glycol-40 hydrogenated castor oil, and mixtures thereof.

Preferably, the skin cleanser contains, based upon the total weight of the skin cleanser, no more than about 6%, and preferably 5%, of the cleaning enhancers for cream formulations and no more than about 2%, and preferably no more than 1% of the cleaning enhancers in thin lotion/milk formulations.

The above described skin cleanser may be prepared by combining the desired components in a suitable container and mixing them under ambient conditions in any conventional mixing means well known in the art, such as a mechanically stirred propeller, paddle, and the like.

Generally, the cleansing composition is topically applied to the affected skin areas in a predetermined or as-needed regimen to bring about improvement, it generally being the case that gradual improvement is noted with each successive application. Insofar as has been determined based upon clinical studies to date, no adverse side effects are encountered. The skin cleanser composition according to the invention may be in the form of a gel, a bath, a wash, a mousse, a shampoo, a rinse, a lotion, a cream, or a spray. The compositions of the present invention may be directed applied to the skin or may be applied onto other delivery implements such as wipes, sponges, brushes, puffs and the like. The compositions may be used in products designed to be left on the skin, wiped from the skin, or rinsed off of the skin.

The skin cleansing compositions according to the invention can be used as a facial cleanser or as a full body cleanser. For example, the skin cleanser composition can be applied to the face, legs, thighs, arms, breasts or any other area of the skin in which mildness is desired. Surprisingly, the cleanser compositions of the invention may be used in products designed to be rinsed off of the skin even though the alkanolamine is water soluble. Indeed, it has been discovered that the anti-inflammatory benefit can be achieved after contact with the skin for as little as one minute and then rinsed with water.

In another embodiment, the invention relates to a method for ameliorating redness or inflammation of mammalian skin by topically applying a composition comprising:
(b) an effective amount of at least one alkanolamine;
(b) a cleansing surfactant; and
(c) water;
wherein said alkanolamine has the following general formula: 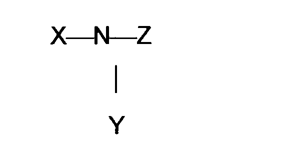 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

The methods according to the invention can be used to treat a variety of skin conditions which result in inflammation or erythema. For example, inflammation or erythema can result from external causes such as sun or wind burn or irritating soaps or cleansers. It is also known that inflammation and erythema can be caused from inherent conditions such as rosacea, atopic dermatitis, or allergic skin reactions. The method according to the invention can be used to treat inflammation and/or erythema caused by both external and inherent conditions.

It has also been discovered that the compounds described above are effective in reducing the skin irritation caused by application of an irritating active ingredient. Accordingly, in another embodiment, the invention relates to a method for ameliorating the irritating effects of a skin irritating composition comprising a cleansing surfactant. The method comprises adding to said composition an effective amount of at least one alkanolamine as described above. The irritating effects of compositions containing an irritating ingredient, such as for example, benzoyl peroxide, alpha-hydroxyacids and derivatives thereof, salicylic acid, surfactants, soaps, natural plant extracts, sunscreen actives, urea, preservatives, and retinoids, such as, retinol, retinyl palmitate, retinoic acid, retinal, and retinyl propionate, can be dramatically reduced upon the incorporation of at least one alkanolamine as described above. One can envision a wide variety of therapeutic agents that are beneficial for the skin yet can cause short term inflammation that would benefit from the current method for ameliorating redness and/or inflammation.

The advantages of the invention and specific embodiments of the skin care compositions prepared in accordance with the present invention are illustrated by the following examples. It will be understood, however, that the invention is not confined to the specific limitations set forth in the individual examples, but rather defined within the scope of the appended claims.

### EXAMPLE 1

The following formula was made in accordance with the teachings of this invention.

**Table 1:**

| **INGREDIENT:** | **WEIGHT PERCENT**: |
|---|---|
| Purified Water | 47.31 |
| Tetrasodium EDTA | 0.09 |
| Glycerine | 6.60 |
| Glycereth-7 | 1.88 |
| Sodium Cocoyl Sarcosinate | 4.71 |
| Decyl Glucoside | 7.07 |
| Ammonium Laureth Sulfate | 7.07 |
| Cocamide DEA | 1-88 |
| Cocamidopropyl Betaine | 7.07 |
| Lauryl Glucoside | 9.42 |
| PEG-120 Methyl Glucose Dioleate | 0.57 |
| Glycol Stearate | 0.94 |
| Citric Acid | 0.06 |
| DMDM Hydantoin | 0.19 |
| Fragrance Roure X5278C | 0.09 |
| | |
| DMAE | 3.00 |
| Glycolic Acid | 1.20 |
| Citric Acid | 0.84 |
| Total | 100 |

The sample was prepared by ad-mixing DMAE to a commercially prepared cleanser containing the above ingredients and adjusting the pH to 8.5 with glycolic acid and citric acid in proportions indicated.

Sixteen subjects were recruited into a clinical evaluation based on a persistent reactive skin history This syndrome involves stress-related flare-ups and exhibits an inflammatory component that leads to the major sign of red, blotchy, irritated skin. The condition can gain a bacterial component leading to infection and symptoms of Rosacea. Due to these components the skin is often sensitive and painful to touch. Consequently, many patients eliminate the use of cleansers and moisturizers exacerbating the condition.

The subjects were evaluated over a 1 week time period to establish the baseline clinical condition and irritation score. The subjects were given the facial cleanser described in Table 1 and asked to use the cleanser twice a day for a 1 week period. A mild moisturizer was added to the skin treatment regiment after the initial 1 week use period, and both were used for an additional period of 3 weeks

Clinical grading scores of the quality of the skin were assessed by a dermatologist based on composite score of skin redness, dryness, roughness, irritation, and rash lesions at baseline and at 1 week intervals. Surprisingly, the use of the cleanser alone for 1 week reduced the reactive skin score by 40%. Continued use of the cleanser containing DMAE over an additional 3 weeks further reduced the reactive skin score to a total of 78% reduction of the clinical symptoms (see Figure 1). Subject self-assessments also reflected a 50% reduction in reactive and inflammation symptoms. Fourteen of sixteen subjects noted at reduction of symptoms equivalent to 1-2 clinical grade points. For these subjects, cleansers are typically associated with increased skin reactiveness and irritation, and may be at best non-inflammatory. It is highly unexpected that a cleanser, with addition of an alkanolamine, i.e., DMAE known itself to be inherently irritating and inflammatory, should in fact reverse the pre-existing reactive skin condition to return it to a healthier and non-reactive and non-inflammed condition.

### EXAMPLE 2

The following formula was made in accordance with the teachings of this invention.

**Table 2:**

| **INGREDIENT:** | **WEIGHT PERCENT**: |
|---|---|
| Purified Water | 67.90 |
| PEG-8 | 8.00 |
| Disodium Lauroamphodiacetate | 5.00 |
| Cetyl Alcohol | 5.00 |
| Glycolic Acid 70% | 4.30 |
| DMAE | 3.00 |
| Glycerin | 2.00 |
| PEG-80 Sorbitan Laurate | 2.00 |
| Xanthan Gum | 1.00 |
| Sodium Cocoyl Glutamate | 0.50 |
| Allantoin | 0.25 |
| Bisabolol | 0.25 |
| Soybean (Glycine Soja) Seed Extract | 0.10 |
| Phenoxyethanol | 0.30 |
| Methylparaben | 0.20 |
| Propylparaben | 0.10 |
| Fragrance, Quest # 32285 | 0.10 |
| | 100.00 |
| | |
| | |
| | |

**Procedure:** Phase A Preparation: Water was added to a kettle and then xantham gum was added and mixed. The mixture was heated to 70-75C. While heating PEG-80 Sorbitan Laurate and Sodium Cocoyl Glutamate was added. At 70-75C Cetyl Alcohol was added and the mixture was mixed for 5 minutes. PEG-8, Methyl Paraben, and Propylparaben were added and then the mixture was mixed completely and then cooled to 55-60C.

In a separate vessel (Phase B), glycerin, Allantoin and Soybean Seed extract were added and mixed. The mixture was then heated to 55C and then cooled to 45C. At 45-50C glycolic acid and DMAE were added and mixed.

Phase B was added to Phase A with mixing. Phenoxyethanol, Bisabolol, Disodium Lauroamphodiacetate, and Fragrance were then added and the mixture was cooled to 35-37C.

### Example 3

The following formula was made in accordance with the teachings of this invention.

**Table 3:**

| **INGREDIENT**: | **WEIGHT PERCENT:** |
|---|---|
| Purified Water | 36.2 |
| Guar Hydroxypropyltrimonium Chloride | 0.25 |
| Tetrasodium EDTA | 0.05 |
| Sodium Trideceth Sulfate (and) Sodium Lauroamphoacetate. | 40.5 |
| Cetearyl Alcohol | 1 |
| Soy Dimethicone Blend 926-149 | 5 |
| 50% Citric Acid Solution | 2 |
| DMAE | 3 |
| Glycolic Acid | 4 |
| Lauramine Oxide | 5 |
| Ammonium Chloride | 2.5 |
| DMD Hydantoin | 0.5 |
| | 100 |
| Purified Water | 36.2 |
| Guar Hydroxypropyltrimonium Chloride | 0.25 |
| Tetrasodium EDTA | 0.05 |
| Sodium Trideceth Sulfate (and) Sodium Lauroamphoacetate. | 40.5 |
| Cetearyl Alcohol | 1 |
| Soy Dimethicone Blend | 5 |
| 50% Citric Acid Solution | 2 |
| DMD Hydantoin | 0.5 |
| Total | 100 |

Procedure: Tetrasodium EDTA was dissolved in water. With agitation, Guar Hydroxypropyltrimonium Chloride was added. The mixture was mixed until dispersed. With agitation, Sodium Trideceth Sulfate and Sodium Lauroamphoacetate were blended into the system. The mixture was warmed to 65C with agitation. Cocamide MEA and Cetearyl Alcohol were blended with agitation into the heated system and mixed until uniform. The system was cooled to 50-55C with agitation. Soy Dimethicone Blend was slowly blended with agitation into system and then mixed until uniform. The system was cooled to 35C. The pH was adjusted with approximately 1 % of 50% Citric Acid solution to pH 6-7. DMAE was blended in with agitation and then mixed until uniform. Then glycolic acid was blended in with agitation and mixed until uniform. In a separate vessel, ammonium chloride was dissolved in water. Salt solution was added to the main system and mixed until uniform. The pH was adjusted to 5.3 to 5.8 with Citric Acid Solution (50%) as needed. Slowly add DMDM Hydantoin into the system, mix until uniform.

### EXAMPLE 4

The following formula was made in accordance with the teachings of this invention.

**Table 4:**

| **INGREDIENT**: | **WEIGHT PERCENT**: |
|---|---|
| Purified Water | 36.2 |
| Guar Hydroxypropyltrimonium Chloride | 0.25 |
| Tetrasodium EDTA | 0.05 |
| Sodium Trideceth Sulfate (and) Sodium Lauroamphoacetate. | 40.5 |
| Cetearyl Alcohol | 1 |
| Soy Dimethicone Blend 926-146 | 5 |
| 50% Citric Acid Solution | 2 |
| Dimethyl MEA | 3 |
| Glycolic Acid | 4 |
| Lauramine Oxide | 5 |
| Ammonium Chloride | 2.5 |
| DMD Hydantoin | 0.5 |
| | 100 |
| Purified Water | 36.2 |
| Guar Hydroxypropyltrimonium Chloride | 0.25 |
| Tetrasodium EDTA | 0.05 |
| Sodium Trideceth Sulfate (and) Sodium Lauroamphoacetate. | 40.5 |
| Cetearyl Alcohol | 1 |
| Soy Dimethicone Blend | 5 |
| 50% Citric Acid Solution | 2 |
| DMD Hydantoin | 0.5 |
| Total | 100 |

Procedure: Tetrasodium EDTA was dissolved in water. With agitation, Guar Hydroxypropyltrimonium Chloride was added and mixed until dispersed. With agitation, Sodium Trideceth Sulfate (and) Sodium Lauroamphoacetate were blended into the system. The mixture was warmed to 65C with agitation. With agitation Cocamide MEA and Cetearyl Alcohol were blended into heated system and mixed until uniform. The system was cooled to 50-55C with agitation. With agitation, Soy Dimethicone Blend was slowly blended into system and mixed until uniform. The system was cooled to 35C. The pH was adjusted with approximately 1% of 50% Citric Acid solution to pH 6-7. With agitation, Dimethyl MEA was blended in an mixed until uniform. With agitation, glycolic acid was blended in and mixed until uniform. In a separate vessel, ammonium chloride was dissolved in water. The salt solution was blended into the main system and mixed until uniform. The pH was adjusted to 5.3 to 5.8 with Citric Acid Solution (50%) as needed. DMDM Hydantoin was slowly added into the system and mixed until uniform.

### EXAMPLE 5: COMPARATIVE EXAMPLE

**Table 5:**

| **INGREDIENT:** | **WEIGHT PERCENT**: |
|---|---|
| | |
| | |
| Purified Water | 59.5 |
| Guar Hydroxypropyltrimonium Chloride | 0.25 |
| Tetrasodium EDTA | 0.05 |
| Sodium Trideceth Sulfate (and) Sodium Lauroamphoacetate. | 29.2 |
| Cetearyl Alcohol | 1.5 |
| Cocamide MEA | 0.5 |
| Soy Dimethicone Blend | 5 |
| 50% Citric Acid Solution | 1 |
| Lauramine Oxide | 0 |
| Ammonium Chloride | 2.5 |
| DMDM Hydantoin | 0.5 |
| | |
| Total | 100 |

Procedure: Tetrasodium EDTA was dissolved in water. With agitation, Guar Hydroxypropyltrimonium Chloride was added and mixed until dispersed. With agitation, Sodium Trideceth Sulfate (and) Sodium Lauroamphoacetate was blended into the system. The mixture was warmed to 65C with agitation. With agitation Cocamide MEA and Cetearyl Alcohol were blended into heated system and mixed until uniform. The system was cooled to 50-55 with agitation. With agitation, Soy Dimethicone was blended into the system and mixed until uniform. The pH was adjusted with approximately 1% of 50% Citric Acid solution. In a separate vessel, ammonium chloride was dissolved in water. Salt solution was blended into the main system and mixed until uniform. The pH was adjusted to 5.3 to 5.8 with Citric Acid Solution (50%) as needed. DMDM Hydantoin was slowly added into the system and mixed until uniform.

Clinical evaluations were conducted on Examples 2, 3, 4, and 5 to evaluate and confirm the ability of DMAE in cleansers to be mild to subjects with hyper-reactive skin. The cleansers were applied to occlusive skin patches and taped to the skin of hyper-reactive subjects for two consecutive 24 hour periods. Two days after removal of the patches the redness (delta a* parameter) of each of the patch sites was measured with a Minolta Chromameter using the conventional L*a*b scale of color evaluation. The redness score (a*) was significantly lower for Examples 2, 3, and 4 with virtually no inflammation noted at these treatment sites compared with the comparative example (Example 5) which did not contain the 3% DMAE ingredient (p<0.05).

Example 2 was tested on 26 subjects with clinically diagnosed rosacea over a 3 week period. Rather than exacerbating the clinical symptoms of rosacea, the cleanser containing DMAE was shown to be well tolerated by 80% of the subjects, improving the skin look and feel of 78% of the subjects, and actually reducing the clinical redness of 75% of the subjects. Surprisingly, the DMAE addition to a cleanser, rather than increase inflammatory potential of the cleanser, reduces the inflammatory nature of the cleanser and is compatible and therapeutic for sensitive, reactive, and inflamed skin.

Having described the invention with reference to particular compositions, theories of effectiveness, and the like, it will be apparent to those of skill in the art that it is not intended that the invention be limited by such illustrative embodiments or mechanisms, and that modifications can be made without departing from the scope or spirit of the invention, as defined by the appended claims. The claims are meant to cover the claimed components and steps in any sequence which is effective to meet the objectives there intended, unless the context specifically indicates the contrary.

## Claims

1. A method of simultaneously cleansing the skin and reducing skin sensitivity and/or skin reactivity comprising topically applying a skin cleanser composition comprising:
(a) an effective amount of at least one alkanolamine of the formula: 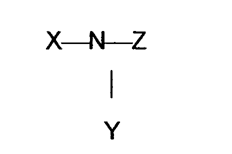 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group;
(b) a cleansing surfactant; and
(c) water.

2. The method according to claim 1, wherein said alkanolamine is selected from the group consisting of ethylaminoethanol, methylaminoethanol, dimethylaminoethanol-amine, isopropanolamine, triethanolamine, isopropanoldimethylamine, ethylethanolamine, 2-butanolamine, choline and serine.

3. The method according to claim 2, wherein said alkanolamine is dimethylaminoethanol.

4. The method according to claim 1, wherein said alkanolamine is present in an amount of from about 0.1 to about 10% by weight of the composition.

5. The method according to claim 4, wherein said alkanolamine is present in an amount of from about 1 to about 5% by weight of the composition.

6. The method of claim 1, wherein the cleansing surfactant is selected from the group consisting of non-ionic surfactants, cationic surfactants, amphoteric surfactants, anionic surfactant, and mixtures thereof.

7. The method of claim 6, wherein the cleansing surfactant is selected from the group consisting of sodium cocoyl sarcosinate, decyl glucoside, lauryl glucoside, ammonium laureth sulfate, cocoamidopropyl betaine, lauryl betaine, sodium cocoamphoacetate, and mixtures thereof.

8. The method of claim 6, wherein the cleansing surfactant is selected from the group consisting of sucrose cocoate, sucrose stearate and mixtures thereof.

9. The method of claim 1, wherein the cleansing composition further comprises a non-ionic emulsifier cleansing enhancer.

10. The method of claim 9, wherein the non-ionic emulsifier cleansing enhancer is selected from the group consisting of isoceteth 20, oleth-2, mixture of PEG-40 hydrogenated castor oil and trideceth-9 , Poloxamer 184, laureth-4, sorbitan trioleate, polyoxyethylene-(2) oleyl ether, sorbitan stearate, cetearyl glucoside, glyceryl oleate, glucamate SSE-20, Glucate DO and mixtures thereof.

11. The method of claim 1 wherein the skin cleanser composition is in the form of a gel, a bath, a wash, a mousse, a shampoo, a rinse, a lotion, a cream, or a spray.

12. The method of claim 1 wherein the skin cleanser composition is incorporated into material carrier selected from a wet wipe, a puff, a brush, or a sponge.

13. A method for ameliorating redness or inflammation of mammalian skin by topically applying a composition comprising:
(c) an effective amount of at least one alkanolamine;
(b) a cleansing surfactant; and
(c) water;
wherein said alkanolamine has the following general formula: 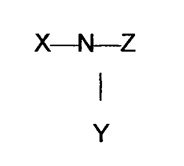 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

14. A method according to claim 13, wherein said composition is applied to red or inflamed skin.

15. A method according to claim 13, wherein said composition is applied to sun burned skin, wind burned skin, or skin that is red or inflamed due to contact with irritating soaps or cleansers.

16. A method according to claim 14, wherein said composition is applied to skin that is red or inflamed due to rosacea, atopic dermatitis, or allergic skin reactions.

17. A method for ameliorating the irritating effects of a skin irritating cleansing composition comprising a cleansing surfactant, said method comprising adding to said composition an effective amount of at least one alkanolamine having the following general formula: 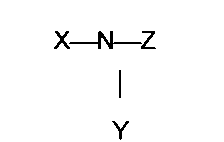 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

18. A method according to claim 17, wherein said alkanolamine is selected from the group consisting of ethylaminoethanol, methylaminoethanol, dimethylaminoethanol-amine, isopropanolamine, triethanolamine, isopropanoldimethylamine, ethylethanol-amine, 2-butanolamine, choline and serine.

19. A method of reducing the irritation of a topical skin treatment comprising topically applying a skin cleanser composition comprising:
(a) an effective amount of at least one alkanolamine of the formula: 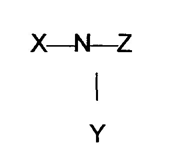 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group;
(b) a cleansing surfactant; and
(c) water;
prior to or intercurrently with application of the irritating topical skin treatment.

20. A method according to claim 17, wherein said alkanolamine is selected from the group consisting of ethylaminoethanol, methylaminoethanol, dimethylaminoethanol-amine, isopropanolamine, triethanolamine, isopropanoldimethylamine, ethylethanol-amine, 2-butanolamine, choline and serine.
